# EUROPEAN PATENT APPLICATION

(11) **EP 2 767 245 A1**
(43) Date of publication of application: **20.08.2014**
(21) Application number: 12840053.8
(22) Date of filing: 09.10.2012
(51) Int. Cl.: A61B 17/68, A61F 2/30, A61L 27/56, A61L 27/58

(54) **DEVICE FOR ATTACHING A MACROPOROUS MATERIAL FOR REGENERATING ARTICULAR CARTILAGE**

(30) Priority: 10.10.2011 ES 201131625
(71) Applicant: Universitat Politècnica de València, 46022 Valencia (ES); Universitat De València, Estudi General, 46010 València (ES); Fundació Privada Institut de Recerca de L'Hospital de La Santa Creu i Sant Pau, 08025 Barcelona (ES)
(72) Inventor: CARDA BATALLA, Carmen, E-46010 Valencia (ES); GALLEGO FERRER, Gloria, E-46022 Valencia (ES); GÓMEZ RIBELLES, José Luis, E-46022 Valencia (ES); MONLLAU GARCÍA, Joan Carles, E-08025 Barcelona (ES)
(74) Representative: Cueto, Sénida
(86) International application number: PCT/ES2012/070700
(87) International publication number: WO 2013/053966

(57) **Abstract**

The invention relates to a device for attaching a macroporous material for regenerating articular cartilage to the subchondral bone, characterised in that it includes: a) a sponge which comprises a biocompatible macroporous material that is suitable for regenerating articular cartilage, b) an attachment system suitable for attaching the device to the subchondral bone, and c) a supporting piece suitable for connecting the sponge to the attachment system. The device according to the invention enables the regeneration of articular cartilage, providing a good system for attachment to the chondral bone, while avoiding damage to the sponge material when attaching the device to the bone.

## Description

### Field of the technique

The present invention relates to a device for attaching a macroporous material to the subchondral bone, said material being biocompatible with a function to regenerate the articular cartilage. More specifically, the invention refers to a device for filling a condral defect with a material, said material being mounted on a fixation system to the bone, such as for example, a screw, nail, a pin or a staple, promoting cartilage regeneration.

### State of the art

The articular cartilage has a very limited reparing capacity due to low cellularity and absence of vascularization. Traumatic articular defects, osteochondritis, degenerative joint diseases such as osteoarthritis or rheumatoid arthritis lead to severe cartilage damage usually associated with pain, loss of mobility, stiffness and progressive joint destruction

Spontaneous cartilage regeneration occurs only when the chondral defect reaches the subchondral bone, and a pathway by which pluripotent cells from bone marrow reach the site of injury from the subchondral bone, is created. Some surgical techniques are aimed at stimulating the regenerative ability of the bone marrow mesenchymal cells, for example, those such as microfracture, whereby the subchondral bone is drilled below the injury in order that the resulting bleeding forms a clot in the area of the chondral defect. This clot will be invaded within days by mesenchymal cells, initiating the formation of a cartilaginous tissue. However, this tissue is normally fibrocartilage tissue instead of hyaline cartilage which the one characteristic of the joint. The newly formed tissue does not have adequate mechanical strength and quickly degenerates. Another surgical technique is osteochondral mosaicplasty whereby cylinder portions of the joint that bear a low load are removed and are transplanted into the defect site seeking to cover it at least 85% of its surface. The problem with this technique is obviously the damage in the donor site and the fact that the properties of cartilage at the extraction site are probably not the same as those needed in the damaged site. This is due to passing from a non loaded area to a loaded area.

More recent tissue engineering techniques have allowed the combined use of synthetic materials and cells. One approach has employed microfracture techniques with the implant of the support material free of cells, so that the cells that produce regenerated tissue are mesenchymal cells coming from the subchondral bone of the patient himself. Said cells invade one scaffold, sponge of biocompatible highly porous mterial that is placed at the defect site. Other approaches have used previously expanded ex-vivo autologous chondrocytes or mesenchymal cells from bone marrow or fat, that are sown on a scaffold that is placed in the defect site in the hope that the implanted cells produce cartilaginous tissue while the synthetic scaffold is resorbed.

Despite the progress made in terms of the identification of the mechanisms leading to the differentiation of mesenchymal cells or ex vivo expanded chondrocytes, to the phenotype of the chondrocytes of hyaline cartilage, one cannot ensure nowadays that tissue regenerated by either of these cell types does not degenerate over time into fibrocartilage, as indeed happens in practice in a significant number of cases with any of the available techniques.

Currently the application of tissue engineering techniques typically presents problems due to the poor fixation of the synthetic scaffold at the site of the defect. The chondral defect is irregular and any material placed on its surface comes off easily. Different products on the market today involve osteochondral implants having a porous portion of ceramic or polymeric material entering the subchondral bone, and that is to be invaded by bone tissue, and another part that must be occupied by the regenerated cartilaginous tissue. The implant of each of these cylinders, of more than 10 mm in height, requires a deep bore in the subchondral bone. The implant, with a diameter slightly bigger than the bore diameter, is placed under pressure afterwards.

### Description of the invention

The present invention provides a device for fixing a material suitable for the regeneration of articular cartilage to subchondral bone characterized by comprising:
a) A sponge comprising a biocompatible macroporous material suitable for articular cartilage regeneration,
b) A atttachment system suitable for securing the device to the subchondral bone, and
c) A supporting piece suitable for attaching the sponge to the atttachment system.

The sponge comprised in the device of the present invention is formed by a very porous and generally, weak material. To avoid that the effort necessary to secure the device to the bone, damages the sponge of the biocompatible macroporous material suitable for the regeneration of articular cartilage, the device of the present invention comprises a supporting piece which is solidly joined to said sponge, allowing its binding to the atttachment system.

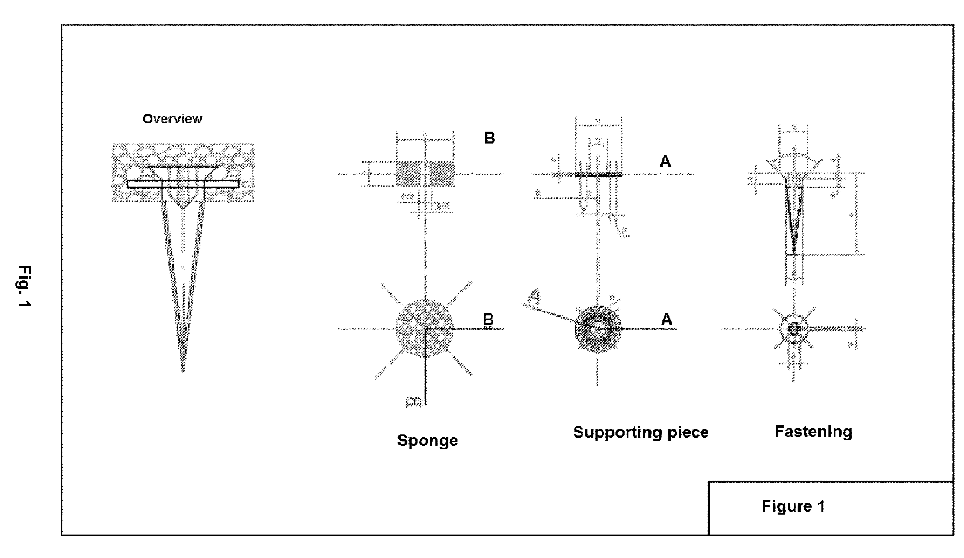

In a preferred embodiment, the supporting piece is located inside the sponge. Preferably, the supporting piece is located at a minimum distance of 0.5 mm and a maximum distance of up to 50% of the thickness of the sponge, with regard to the sponge base. The distance of the supporting piece to the sponge base depends on the mechanical characteristics of the material used in the manufacture of the sponge, in such a way that, once the device is placed, the part in contact with the subchondral bone is the sponge (see figures 1, 2 and 3).

The supporting piece, as defined in this application, consists mainly of a material capable of withstanding mechanical stresses. Additionally, this piece is characterized by having holes of sufficient size to permit blood and stem cells flowing between the subchondral bone and the sponge, thus allowing regeneration of articular cartilage.

Thus, in another preferred embodiment, the device of the present invention is characterized in that the supporting piece is formed mainly by a non-porous material, and in that it has holes of a diameter greater than 500 µm. These holes are partially filled with the same macroporous material comprised in the sponge such as defined in this patent application. These holes allow the continuity between the regenerated tissue inside the sponge and the bone from where he blood and mesenchymal cells have to originate.

Preferably, the non-porous material which is the main constituent of the supporting piece suitable for attaching the sponge to the atttachment system device of the present invention, is a partially bioresorbable or bioresorbable material. Still more preferably, said non-porous material is selected from the group consisting of polylactic acid, a copolymer of polylactic acid / polyglycolic acid, polycaprolactone, polyvinyl alcohol and mixtures thereof.

Preferably, the non-porous material comprised in the supporting piece, as defined in this application, is a bio-stable material. Still more preferably, said non-porous material is selected from the group consisting of n-alkyl polyacrylate, n-alkyl polymethacrylate and mixtures thereof.

In another preferred embodiment, the device of the present invention is characterized in that the supporting piece suitable for attaching the sponge to the atttachment system, has a ring shape. Preferably, the supporting piece has the shape of a flat ring.

In another preferred embodiment, the device of the present invention is characterized in that the atttachment system suitable to secure the device to the subchondral bone, and the sponge that comprises a biocompatible macroporous material suitable for articular cartilage regeneration, can move independently from each other. Preferably, the atttachment system and sponge, as defined in this patent application, can rotate and slide independently.

In another preferred embodiment, the device for fixing a material suitable for the regeneration of articular cartilage, to the subchondral bone of the present invention, is characterized in that the atttachment system and the supporting piece, as defined in this patent application, are a single piece. In this case, the device of the invention is also characterized in that the atttachment system and the sponge can not move independently from each other.

In another preferred embodiment, the device for fixing a material suitable for the regeneration of articular cartilage to the subchondral bone, as described in this patent application, is characterized in that the atttachment system comprises one or more fixing elements independently selected from the group consisting of a screw, a nail, a pin and a staple.

Preferably, the device of the present invention may comprise more than one fastening point. Thus, the device of the present invention allows to fill the chondral defect using devices with a single fastening element along with other devices comprising several fastening elements, wherein said fastening elements may be identical or different from each other.

When the atttachment system to the subchondral bone comprises a screw, a torque is required to secure the screw to the bone. If the screw and the sponge were solidly united, the torque directly exerted on the sponge could damage the biocompatible macroporous material suitable for the regeneration of articular cartilage comprised in said sponge.

Similarly, when the atttachment system to the subchondral bone comprises a nail, a pin or a staple, one needs to use an impactor, that in case of being directly applied onto the sponge, for its attachment to the bone, it could also damage the sponge.

In contrast, in the device of the present invention the torque or impaction takes place on the supporting piece instead of on the sponge, thus avoiding that the biocompatible macroporous material comprised in the sponge to be damaged.

Furthermore, when the atttachment system comprises at least one screw and said atttachment system does not form a single piece with the supporting piece, the device of the invention allows free movement between the atttachment system and the sponge. Consequently, the supporting piece allows the screw to rotate without the sponge rotating, further decreasing the possibility for the sponge to be damaged.

In an even more preferred embodiment, the device of the present invention is characterized in that when the attachment system suitable for fixing the device to subchondral bone comprises at least a screw, it can rotate independently of the sponge. Thus, the device of the invention allows to tighten the screw to the bone without the torsion damaging the sponge.

In another preferred embodiment, the device as described in this patent application, is characterized in that the atttachment system suitable to secure the device to the subchondral bone is a metallic atttachment system or an attachment system mainly formed by a bioresorbable or partially bioresorbable material.

Preferably, the metallic atttachment system comprises titanium or porous tantalum.

Preferably, if the atttachment system is mainly made of a bioresorbable or partially bioresorbable material, this is the same material comprised by the sponge. Preferably, if the the atttachment system is bioresorbable or partially bioresorbable, independently on whether the material is the same as the sponge material or not, the system comprises a material selected from the group consisting of polylactic acid, a copolymer of lactic acid / polyglycolic acid, polycaprolactone, polyvinyl alcohol and mixtures thereof.

Different architectures and different pore of bioresorbable and bio-stable materials have been successfully tested as sponge material. Architectures of interconnected pores (spherical, cylindrical, heterogeneous forms) with average sizes between 50 and 400 microns which include pores have been successfully tested. The materials successfully employed were those pertaining to the family of naturally occurring polysaccharides, proteins and synthetic polymers.

In another preferred embodiment, the device described in this patent application is characterized in that the biocompatible macroporous material comprised in the sponge is a partially bioresorbable or bioresorbable material.

Preferably, when the material comprised in the sponge is a partially bioresorbable or birebsorbable material, so is the material forming the supporting piece and the atttachment system. Still more preferably, the sponge, the atttachment system and the supporting piece comprise the same partially bioresorbable or bioresorbable material.

Preferably the bioresorbable or partially bioresorbable material, such as described above, is selected from the group consisting of polylactic acid, a copolymer of polylactic acid / polyglycolic acid, polycaprolactone, polyvinyl alcohol and mixtures thereof.

The sponge can be produced for example by a method of solid - liquid phase separation in the presence of a porogen, as described in M. Lebourg, J. Suay Ant6n, J.L. Gómez Ribelles Hybrid structure in PCL-HAp scaffold resulting from biomimetic apatite growth. Journal of Materials Science. Materials in Medicine 21 (1) 33-44 (2010). The device obtaining process is carried out in a mold in which the atttachment system and the supporting piece, both previously manufactured, are placed. After removal of solvents and porogen, the sponge and the supporting piece are solidly united, and the atttachment system is included in the sponge with ability to rotate or slide with respect to the supporting piece.

Similarly the device can be manufactured by introducing into a mold, in which the atttachment system and the supporting piece in the right position have been previously placed, the polymer to make the sponge in powder form, mixed with the porogen. The assembly is heated until the polymer melts, and upon cooling, the porogen is extracted with suitable solvents.

In another preferred embodiment, the device described in this patent application is characterized in that the biocompatible macroporous material comprised in the sponge is a bio-stable material.

Preferably, the bio-stable material comprised in the sponge is the same as the material comprised in the supporting piece for joining the sponge with the atttachment system, as described in this patent application.

In an even more preferred embodiment, when the material comprised in the sponge and in the supporting piece is the same bio-stable material, the atttachment system is an atttachment system of metallic fixing, as defined in this patent application.

Additionally it is preferred that the bio-stable material, as described in the preceding paragraphs, is selected from the group comprising an n-alkyl polyacrylate, n-alkyl polymethacrylate, and mixtures thereof.

The polymers of the family of n-alkyl polyacrylates or n-alkyl polymethacrylates may be more or less hydrophilic, for example, copolymers of ethyl acrylate and hydroxyethyl acrylate in different proportions. These materials are good candidates because they have been used in other biomedical applications as permanent implants: bone cements, filling for vertebrae, infraocular lenses.

In this case the porous structure of the sponge can be prepared, when the atttachment system is made of metal, from a template, as described in: R. Brigido Diego, Olmedilla M. Pbrez, A. Serrano Aroca, JL Ribelles G6mez, M. Monleon Pradas, G. Gallego Ferrer, M. Salmerón Sánchez Acrylic scaffolds with interconnected spherical pores and controlled hydrophilicity for tissue engineering; J.Mat. Mat Sci. Medicine 16, 693-698 ( 2005); A.J. Campillo - Fernandez, R. E. Unger, K. Peters , S. Halstenberg, M. Santos, M. Salmerón Sánchez, JM Meseguer Duenas, M. Monleón Pradas, J. L. Ribelles Gómez, C. J. Kirkpatrick Analysis of the biological response of endothelial and fibroblast cells cultured on synthetic scaffolds with various hydrophilic / hydrophobic ratios. Influence of fibronectin adsorption and conformation. Tissue Engineering 15, 1331-1341 (2009), including the atttachment system for example, a screw, and the supporting piece, in the mold. Subsequently the sponge material is polymerized in the free space of the template, which is then dissolved using suitable solvents. Thus, the sponge remains solidly connected to the supporting piece.

In another preferred embodiment, the device as described in this patent application, is characterized in that the pores of the material comprised in the sponge are coated with fibrin or a polysaccharide. Preferably, the polysaccharide is hyaluronic acid or chitosan.

The method of coating the inner walls of the sponge, as described in the references [Joao F. Mano, Graham Hungerford, José L. Gómez Ribelles Bioactive poly (L-lactic acid) -chitosan hybrid scaffolds Mat. Sci. Eng. C 28, 1356-1365 (2008), J. Costa Antunes, J.M. Soria, J. Mano, J.L. Gómez Ribelles. Biodegradable Poly (L-lactic acid) scaffolds with internal hyaluronic acid coating. Biological response in vitro. (Abstract) Tissue Engineering: Part A 14, 814 (2008); J.C. Antunes, J.M. Oliveira, R.L. Reis, J.M. Soria, J.L. Gómez Ribelles, J.F. Mano, Novel poly (L-lactic acid) lhyaluronic acid macroporous hybrid scaffolds: Characterization and assessment of citotoxicity Journal of Biomedical Materials Research. Part B- Applied Biomaterials published on line)] can be performed on the entire device, with the sponge already mounted on the atttachment system as described in this patent application.

In another preferred embodiment, the device of this invention, as described in this patent application is characterized in that the sponge comprises additional compounds useful in the regeneration of articular cartilage. Preferably these compounds are autologous expanded chondrocytes or pluripotent cells.

In another preferred embodiment, the device as described in this patent application is characterized in that the sponge may further comprise at least one drug. Preferably, the drug is gradually released in the region of the implant to help in articular cartilage regeneration.

In another preferred embodiment, the device of the present invention as described in this patent application, is characterized in that the supporting piece has at least one slot of a given shape and the sponge has a cross section with the same shape as the slot. Preferably, said slot is cross shaped.

The cross section in the sponge can be made with a sharp blade or die, once the sponge is already well embebded into the device of the invention.

In another preferred embodiment, the device as described in this patent application is characterized in that it further comprises a tool for fixing the system to the subchondral bone.

Preferably, when the atttachment system comprises at least one screw, the tool described in this patent application allows one to screw the screw to the subchondral bone.

Preferably, when the atttachment system comprises at least one nail, a pin or a staple, the tool described in this patent application allows said fastening element to impact the subchondral bone.

Preferably, when the atttachment system is a nail, pin or staple, the supporting piece has a slot with a flat or any other suitable shape, so that a mechanical tool fits in said slot. Said mechanical tool is rod shaped and will impact the atttachment system nailing it to the bone. In this case the sponge has a cross shaped bore or cut in its center that allows the passage of the tool and that will be closed when the tool is removed.

In an even more preferred embodiment, the tool for screwing or impacting the atttachment system in the bone is a rod, preferably made of metal, with at least one end that fits into the slot of the supporting piece. When the tool is suitable for screwing it has a cylindrical shape what permits it to rotate with regard to the sponge without damaging it.

In another even more preferred embodiment, the rod described in this patent application comprises a handle at the end opposite to the one that fits into the slot of the supporting piece of the device of the invention.

This rod is slidable inside a cylinder, preferably also a metallic cylinder, intended to provide support for extracting the inner rod from the supporting piece once the implant is fixed.

The tool length is sufficient to allow intervention by arthroscopy. The entire assembly, including the device mounted on one end of the tool can be sterilized and mounted, and presented - as previously mounted - in a sterile container.

In an even more preferred embodiment, the device of the present invention is characterized in that the macroporous sponge material has sufficient elasticity to close the space left after removal of the tool.

In another even more preferred embodiment, the device of the present invention that comprises a tool for setting, preferably by screwing or impacting, the device to the subchondral bone, as defined in this patent application, is characterized in that it is sterilized.

In another preferred embodiment, the device of the present invention may have different shapes and sizes depending on the chondral defect to be filled.

### Brief Description of the Figures

Figure 1: Device comprising a metallic screw, and sponge and supporting piece made made of a bio-stable material, or biodegradable material wherein the screw and the sponge can rotate freelywith respect to each other. A specific case is described in example 1
Figure 2: Device comprising a nail, a supporting piece and sponge, the sponge made of bioresorbable material, wherein the nail and supporting piece form a single piece solidly united to each other, as in the example 2.
Figure 3: A device comprising several fastening elements and that does not have a cylindrical shape as described in example 3.

### EXAMPLES

### Example 1 - bio-stable system

A device is manufactured wherein the following elements are used: a metallic titanium screw, a porous sponge which is manufactured in a network of copolymer of hydroxyethyl acrylate (HEA) and ethyl acrylate (EA) using ethylene glycol dimethacrylate as cross-linker, and a ring shaped supporting piece of the same material as the sponge.

The supporting ring is first prepared and punched, synthesizing the material for copolymerization of hydroxyethyl acrylate and ethyl acrylate as a sheet or plate of suitable thickness.

In a transparent mold prepared for that purpose the device is prepared by synthesizing the porous sponge wrapping the screw head and the supporting ring, in such a way that the screw can freely rotate inside the support ring and the sponge, as described in figure 1. The mold is cylindrically shaped so that the sponge as synthesized also has this cylindrical shape. The sponge has a height or thickness usually from 3 to 7 mm, according to the thickness of the cartilage to be treated.

The porous sponge is synthesized by setting the metallic screw and the supporting piece inside the mold, both surrounded by polymethylmethacrylate microspheres of 200 microns diameter, said microspheres filling the volume of the mold. The microspheres are sintered at a temperature of 180 ° C and under a slight pressure, forming a template for the porous structure. The mixture of monomers and the crosslinker, initiator of the photosensitive polymerization, are introduced into the mold, in the hollow space left by the porous template. The polymerization is performed under ultraviolet light at room temperature. Subsequently the supporting piece is demolded, repeatedly washed with acetone to remove the porogen template, and any other monomer remains, then it is washed with ethanol, changing the solvent finally and gradually for distilled water. The supporting piece is dried in vacuum, packed into a suitable bag and sterilized by a process suitable to the material of which the implant is made.

The outer diameter of the sponge can be from 3 to 15 mm. As many devices as may be necessary are used for the treatment of an injury or defect in the cartilage, to cover the affected area, analogous to the classical treatment of mosaicplasty.

### Example 2 - bioresorbable system

A device is prepared in which the following componentes are used: a nail made of polylactic acid (PLLA), a porous sponge manufactured in polycaprolactone (PCL) and a ring shaped supporting piece of the same material as the one of the nail. The nail and the supporting ring are manufactured to form a solidly attached single piece.

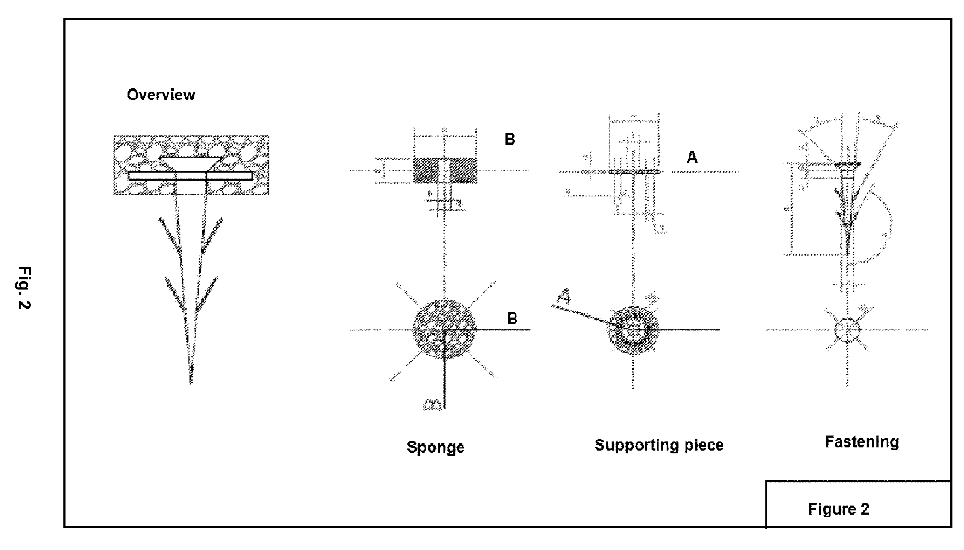

The assembly of the nail and the supporting piece are fixed inside the mold to manufacture the piece, and they are surrounded with a mixture of ethyl polymethacrylate microspheres of 90 microns in diameter and polycaprolactone pulverized to obtain a particle size of about 50 microns. The mold temperature is increased to 65 ° C melting the polycaprolactone under slight pressure. After cooling the mold, the piece is demolded and the porogen is dissolved with repeated washing in ethanol. The dimensions are similar to those describe in Example 1. See Figure 2.

### Example 3 - System for heterogeneous cartilage defect

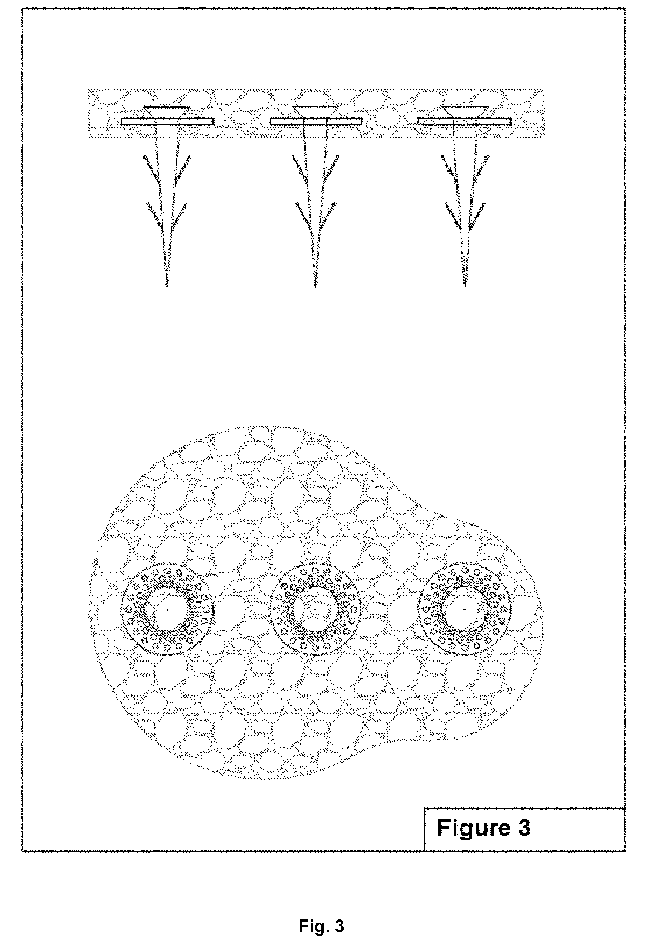

A device as in Example 1 or 2 is prepared, wherein the sponge does not have a cylindrical shape, but is taylor made according to the shape of the cartilage defect or injury to be treated. To this end, a mold for the sponge with the shape of the injury obtained from an image, thomogram, or scan, is prepared. The manufactured device includes as many fasteners with the appropriate type as needed according to the surgeon in charge of the treatment. See Figure 3.

## Claims

1. A device for fastening a material suitable for the regeneration of articular cartilage to the subchondral bone **characterized in that** it comprises:
a) a sponge comprising a biocompatible macroporous material suitable for articular cartilage regeneration,
b) an atttachment system suitable for securing the device to the subchondral bone, and
c) a supporting piece suitable for attaching the sponge to the atttachment system.

2. Device according to claim 1, wherein the supporting piece is mainly formed by a non-porous material and has holes of a diameter greater than 500 µm partially filled with the same macroporous material comprised in the sponge.

3. Device according to any of the preceding claims, wherein the supporting piece has the shape of a flat ring.

4. Device according to any of the preceding claims, wherein the atttachment system and the sponge move independently from each other.

5. Device according to any one of claims 1 to 3, wherein the atttachment system and the supporting piece form a single piece.

6. Device according to any of the preceding claims, wherein the atttachment system comprises one or more fixing elements independently selected from the group consisting of a screw, a nail, a pin and a staple.

7. Device according to any of the preceding claims, wherein the atttachment system is a metallic attachement system or an atttachment system mainly consisting of a bioresorbable or partially bioresorbable material.

8. Device according to claim 7, wherein the metallic atttachment system comprises titanium or porous tantalum.

9. Device according to claim 7, wherein the atttachment system comprising a bioresorbable or partially bioresorbable material is mainly composed of the same material comprised in the sponge.

10. Device according to any of the preceding claims, wherein the macroporous biocompatible material comprised in the sponge is a partially bioresorbable or bioresorbable material.

11. Device according to claim 10, wherein the sponge, the atttachment system and the supporting piece comprise the same bioresorbable or partially bioresorbable material.

12. Device according to any one of claims 9 to 11, wherein the bioresorbable or partially bioresorbable material is selected from the group consisting of polylactic acid, a copolymer of polylactic acid / polyglycolic acid, polycaprolactone, polyvinyl alcohol and mixtures thereof.

13. Device according to any one of claims 1 to 8, wherein the biocompatible macroporous material comprised in the sponge is a bio-stable material.

14. Device according to claim 13, wherein the supporting piece comprises the same bio-stable material as the sponge, and the atttachment system is a metallic atttachment system.

15. Device according to any one of claims 13 or 14, wherein the bio-stable material is selected from the group comprising an n-alkyl polyacrylate, n-alkyl polymethacrylate, and mixtures thereof.

16. Device according to any one of the preceding claims, wherein the pores of the material comprised in the sponge are coated with fibrin or a polysaccharide.

17. Device according to claim 16, wherein the polysaccharide is chitosan or hyaluronic acid.

18. Device according to any of the preceding claims, wherein the sponge comprises expanded autologous chondrocytes or pluripotent cells.

19. Device according to any of the preceding claims, wherein the sponge further comprises at least one drug.

20. Device according to any of the preceding claims, wherein the supporting piece has at least one slot of a specific shape and the sponge comprises a cross section with the same shape as the slot.

21. Device according to any of the preceding claims, wherein the device further comprises a tool for screwing the device to the subchondral bone.

22. Device according to any of the preceding claims, wherein the device further comprises a tool to impact the device onto the subchondral bone.

23. Device according to claim any one of claims 21 or 22, wherein the tool is a rod that fits in the slot of the supporting piece by at least one of its ends.

24. Device according to claim 23, wherein the tool has a handle at the end opposite to the end that fits into the slot of the supporting piece.

25. Device according to any one of claims 21 to 24, **characterized in that** the macroporous material comprised in the sponge has an elasticity sufficient to close the space left after extraction of the tool.

26. Device according to any one of claims 21 to 25, wherein said device is sterilized.
